# EUROPEAN PATENT SPECIFICATION

(11) **EP 2 694 020 B1**
(45) Date of publication and mention of the grant of the patent: **08.05.2019**
(21) Application number: 12712641.5
(22) Date of filing: 29.03.2012
(51) Int. Cl.: A61K 8/27, A61K 8/35, A61Q 11/00

(54) **ORAL CARE COMPOSITIONS**
MUNDPFLEGEZUSAMMENSETZUNGEN
COMPOSITIONS DE SOIN BUCCAL

(30) Priority: 04.04.2011 EP 11160953
(43) Date of publication of application: 12.02.2014
(73) Proprietor: Unilever PLC, London EC4Y 0DY (GB); Unilever N.V., 3013 AL Rotterdam (NL)
(72) Inventor: ALONSO, Coralie, Claudine, Bebington Wirral Merseyside CH63 3JW (GB); GOLDING, Stephen, Bebington Wirral Merseyside CH63 3JW (GB); THORNTHWAITE, David, William, Bebington Wirral Merseyside CH63 3JW (GB)
(74) Representative: Tansley, Sally Elizabeth
(86) International application number: PCT/EP2012/055720
(87) International publication number: WO 2012/136574

(56) References cited:
- EP-A1- 1 508 323
- WO-A2-2007/105071
- WO-A2-2008/042944
- DATABASE WPI Week 201124 Thomson Scientific, London, GB; AN 2011-A46385 XP002668677, & CN 101 904 835 A (GUANGDONG ZHONGDA GREENFIELD BIO-TECH CO) 8 December 2010 (2010-12-08)
- KULANDAISAMY A ET AL: "Synthesis, spectral, redox and biological screening studies of schiff base copper(II), nickel(II), cobalt(II), oxovanadium(II) and zinc(II) complexes derived from curcumin and 4-aminoantipyrine", POLISH JOURNAL OF CHEMISTRY, POLSKIE TOWARZYSTWO CHEMICZNE, PL, vol. 82, no. 3, 1 January 2008 (2008-01-01), pages 469-480, XP009155991, ISSN: 0137-5083
- SONG Y M ET AL: "Syntheses, characterization and biological activities of rare earth metal complexes with curcumin and 1,10-phenanthroline-5,6-dione", JOURNAL OF INORGANIC BIOCHEMISTRY, ELSEVIER INC, US, vol. 103, no. 3, 1 March 2009 (2009-03-01) , pages 396-400, XP025939044, ISSN: 0162-0134, DOI: 10.1016/J.JINORGBIO.2008.12.001 [retrieved on 2008-12-13]

## Description

### Field of the Invention

The present invention is concerned with oral care compositions. More particularly, the present invention is concerned with oral care compositions containing curcumin compounds which are complexed with metal ions.

### Background of the Invention

Curcumin compounds may be used in oral care compositions to exert beneficial physiological effects, such as prevention or amelioration of gingivitis or periodontitis. GB 2317339 discloses oral compositions for prevention and treatment of dental caries, periodontal diseases and other diseases of the oral cavity. The compositions include a curcuminoid and a fluoride ion source, in combination with oral care actives and carrier materials. SU 1,132,945 discloses incorporating extracts of turmeric or ginger into toothpaste compositions for improved anti-inflammatory effect on tissues of the oral cavity and treatment of certain diseases of the mucous membranes of the oral cavity and marginal periodontitis.

CN10 904835 describes a curcumin-zinc compound and for use in a variety of health care products including toothpastes.

Oral care compositions containing zinc salts are disclosed in EP1508323 for preventing or reducing the prevention of bacteria.

The present inventors have found that the complexation of curcumin compounds with metal ions provides improved performance in an oral care context. Specifically, such metal complexes have been found to be particularly effective in suppressing the activity of microbes found in the oral cavity.

### Summary of the Invention

Accordingly, the present invention provides an oral care composition comprising a coordination complex in which a metal cation is complexed to one or more ligands which are derived from one or more curcumin compounds.

### Detailed Description of the Invention

Curcumin is a polyphenolic yellow pigment which occurs naturally in the rhizome of the species *Curcuma longa,* which is grown commercially and sold as turmeric, a yellow-orange dye. It is a member of the linear diarylheptanoid class of natural products in which two oxy-substituted aryl rings are linked together through a seven-carbon chain. Curcumin is also known as diferuloylmethane or (1E,6E)-1,7-bis (4-hydroxy-3-methoxy-phenyl) hepta-1,6-diene-3,5-dione.

The term "curcumin compound" in the context of the present invention means curcumin or a natural or synthetic analogue thereof which is selected from demethoxycurcumin (also known as *p*-hydroxycinnamoyl(feruloyl)methane), or bis-demethoxycurcumin (also known as *p*,*p*-dihydroxydicinnamoylmethane),.

Mixtures of any of the above described materials may also be used.

Curcumin compounds used to form the coordination complex include curcumin, demethoxycurcumin, bis-demethoxycurcumin and mixtures thereof.

The coordination complex used in the present invention comprises one or more ligands which are derived from the one or more curcumin compounds as described above. In such curcumin compounds, two oxy-substituted aryl rings are linked together through a seven-carbon chain, and the seven-carbon chain contains a 1,3-diketone group. The 1,3-diketone group is able to exist either in the keto-enol or β-diketo tautomeric forms. Derivation of the ligand from the curcumin compound typically occurs by dissociation of the enolic proton in solution to give a monoanionic bidentate ligand. This ligand can coordinate to a metal cation through the two oxygen atoms, thereby forming the coordination complex.

Suitable metal cations used to form the coordination complex include any nontoxic metal cation able to form a coordination complex with at least one of the curcumin compounds as described above. Examples of such metal cations include potassium, zinc, calcium, magnesium, selenium, copper, chromium, vanadium, iron, manganese, palladium, gallium, indium, silver, tin and aluminium cations. Of the above metal cations, divalent species are preferred, for example Zn²⁺,Cu²⁺,Mg²⁺,Fe²⁺,Se²⁺ and Sn²⁺, with Zn²⁺ being most preferred. Mixtures of any of the above described metal cations may also be used.

A preferred class of coordination complex for use in the present invention may be described by the general formula (I):

[M(L)ₓ(H₂O)_{y}] (I)

in which M is a metal cation;
x is 1, 2 or 3;
y is 0 or 1, and
L is a curcumin-compound-derived ligand of general formula (II): 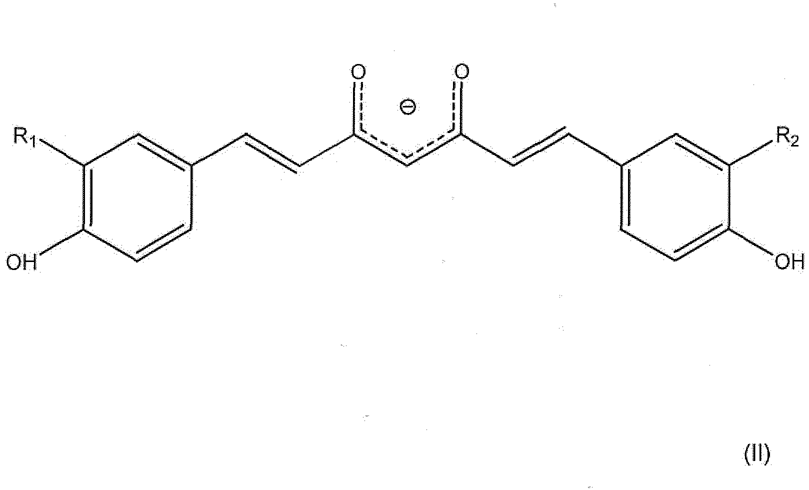 in which R₁ and R₂ are each independently selected from
   -H and -OCH₃.

In particularly preferred complexes of general formula (I), M is a divalent metal cation, most preferably Zn²⁺, x is 1 or 2 and y is 0 or 1.

Mixtures of any of the above described materials may also be used.

### Process & Product Form

A typical process for preparing the oral care composition according to the invention comprises the following steps:
(a) obtaining a coordination complex in which a metal cation is complexed to one or more ligands which are derived from one or more curcumin compounds, and
(b) combining the coordination complex obtained in (a) with an oral care base formulation which is suitable for treating the surfaces of the oral cavity.

Generally, the coordination complex may be obtained by reacting one or more curcumin compounds (as defined above) together with a source of the metal cation, such as a metal salt.

In a typical process used to obtain the coordination complex, a mixture of the curcumin compound and the metal salt is heated to at least 40°C in an appropriate solvent (usually an organic solvent such as C₁₋₃ monohydric alcohol, acetone, or mixtures thereof). The coordination complex typically separates from the mixture as a precipitate which may then be filtered, purified by washing if necessary and dried. The pH of the mixture may be adjusted if necessary in order to aid precipitation of the coordination complex.

For example, a coordination complex of zinc and curcumin may be obtained by mixing a zinc salt (such as zinc acetate) with curcumin in a molar ratio of from 1:1 to 1:5 in an organic solvent (such as ethanol and propyl alcohol), and refluxing at 100°C under nitrogen for 3 to 5 hours. The zinc-curcumin complex precipitates and may be separated by cold filtration.

Alternatively, a complexation method for curcumin with divalent metal cations has been described in the literature which does not require the use of high temperatures or organic solvents. According to this method, curcumin is mechanically mixed together with a metal salt (zinc sulphate, copper sulphate, magnesium sulphate or sodium selenite) in a molar ratio of 1:1. The resulting homogeneous powder is mixed with a glycerol/water solvent and shaken at 25°C. Drying of the resultant paste followed by washing yields the zinc, copper, magnesium or selenium-curcumin complex.

The coordination complex (obtainable as described above) is then combined with an oral care base formulation which is suitable for treating the surfaces of the oral cavity, in order to form the final oral care composition for use according to the invention.

Suitable oral care base formulations may take various product forms. Examples of suitable product forms include dentifrice, mouthwash, tooth powder, chewing gum, lozenge, mouth spray, floss or dental strip.

The amount of coordination complex (as defined above) in the final oral care composition of the invention will depend on the oral care base formulation used, but generally ranges from 0.0005 to 5%, preferably from 0.05 to 3%, more preferably from 0.2 to 2% by total weight coordination complex (as defined above) based on the total weight of the composition.

Preferred oral care base formulations are those which are suitable for brushing and/or rinsing the surfaces of the oral cavity.

Such formulations generally comprise a continuous phase comprising water or monohydric or polyhydric alcohol or a mixture thereof.

Preferably the continuous phase comprises water or polyhydric alcohol or a mixture thereof.

An example of a preferred type of oral care base formulation in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice suitable for use in the invention will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A dentifrice suitable for use in the invention will generally contain further ingredients to enhance performance and/or consumer acceptability such as abrasive cleaning agent, binder or thickening agent, and surfactant.

For example, a dentifrice will usually comprise an abrasive cleaning agent in an amount of from 3 to 75% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Furthermore, the dentifrice will usually contain a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of C₈ to C₁₈ alkyl sulphates (for example sodium dodecyl sulphate), C₈ to C₁₈ alkyl sulphosuccinates (for example dioctyl sodium sulphosuccinate), C₈ to C₁₈ alkyl sulphoacetates (such as sodium dodecyl sulphoacetate), C₈ to C₁₈ alkyl sarcosinates (such as sodium dodecyl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Another example of a preferred type of oral care base formulation in the context of the present invention is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition suitable for use in the invention will usually contain an aqueous continuous phase. The amount of water generally ranges from 70 to 99% by weight based on the total weight of the mouthwash.

A mouthwash composition suitable for use in the invention may also contain a monohydric alcohol such as ethanol, isopropanol or a mixture thereof. If present, the amount of monohydric alcohol typically ranges from 1 to 25%, preferably from 10 to 20% by weight based on the total weight of the mouthwash.

A mouthwash composition suitable for use in the invention will generally contain further ingredients to enhance performance and/or consumer acceptability, such as the humectants and surfactants mentioned above for dentifrices. The amount of humectant generally ranges from 5 to 20% by weight based on the total weight of the mouthwash and the amount of surfactant generally ranges from 0.1 to 5% by weight based on the total weight of the mouthwash.

### Further Optional Ingredients

Oral care base formulations such as the dentifrices or mouthwashes described above may also contain further optional ingredients customary in the art such as fluoride ion sources, anticalculus agents, buffers, flavouring agents, sweetening agents, colouring agents, opacifying agents, preservatives, antisensitivity agents, delivery-enhancing polymers (such as polymers based on a copolymer of methyl ether with maleic anhydride), antimicrobial agents and tooth remineralisation agents, such as hydroxyapatite particles or precursors of crystalline hydroxyapatite such as calcium silicate, calcium sodium phosphosilicate (bioglass), amorphous calcium phosphate and the like.

A preferred class of optional ingredient for inclusion in the oral care compositions described herein includes antioxidant vitamins.

Examples of such antioxidant vitamins include tocopherols and derivatives thereof. The tocopherols are mono, di, and trimethylated derivatives of the parent substance tocol (2-methyl-2-(4,8,12-trimethyltridecyl) chroman-6-ol). The configuration 2R,4'R,8'R is assigned to alpha-tocopherol, which occurs most frequently in nature and is the most important source of vitamin E activity. It is occasionally also called RRR-alpha-tocopherol. The tocopherols and derivatives thereof which are preferred for use in the invention are alpha-tocopherol and its esters, such as alpha-tocopherol succinate, alpha-tocopherol nicotinate and alpha-tocopherol acetate. Alpha-tocopherol acetate is especially preferred. Mixtures of any of the above described materials may also be used.

When present, the amount of tocopherols and/or derivatives thereof suitably ranges from 0.0001 to 10%, preferably from 0.01 to 5%, more preferably from 0.5 to 1.5% by total weight tocopherols and/or derivatives thereof based on the total weight of the oral care composition.

Further examples of such antioxidant vitamins include ascorbic acid and/or derivatives thereof. The ascorbic acid and/or derivatives thereof for use in the present invention may be ascorbic acid and/or any cosmetically acceptable watersoluble or oil-soluble ascorbic acid derivative. The term "ascorbic acid and/or derivatives thereof" encompasses ascorbic acid as well as esters of ascorbic acid, and ester salts of ascorbic acid such as ascorbyl phosphates as well as ascorbic acid derivatives such as ascorbyl palmitate, ascorbyl tetraisopalmitate (for example available from DSM), ascorbyl dipalmitate (for example, NIKKOL CP available from Nikko Chemical), ascorbyl linoleate, ascorbyl octanoate, 2-O-D-glucopyranosyl-L-ascorbic acid, which is an ester of ascorbic acid and glucose and usually referred to as L-ascorbic acid 2-glucoside or ascorbyl glucoside, and its metal salts. The term "ascorbyl phosphate" denotes metal salts of mono- and polyphosphoric acid esters of ascorbic acid in which the phosphorylated hydroxy group of the ascorbic acid molecule features one or more phosphoric acid (phosphate) units, and in which metal cations (such as sodium and/or magnesium or calcium ions) are also present. The term "poly" generally denotes 2 to 10, preferably 2 to 4 phosphate units. The ascorbyl phosphates may also be referred to in general as "ascorbyl (poly)phosphates" to embrace both mono- and polyphosphates. Typical ascorbyl phosphates for use in the present invention are L-ascorbic acid phosphate ester salts such as sodium ascorbyl phosphate, potassium ascorbyl phosphate, magnesium ascorbyl phosphate, calcium ascorbyl phosphate and sodium magnesium L-ascorbyl-2-monophosphate. Commercially available ascorbyl phosphates comprise trisodium L-ascorbyl-2-monophosphate which is available as STAY-C®50 form DSM Nutritional Products AG, (4303 Kaiseraugst, Switzerland) and magnesium L-ascorbyl phosphate (available from Showa Denko) and sodium magnesium L-ascorbyl-2-monophosphate. The preferred ascorbyl phosphate for the purposes of the present invention is trisodium L-ascorbyl-2-monophosphate. Mixtures of any of the above described materials may also be used.

When present, the amount of ascorbic acid and/or derivatives thereof suitably ranges from 0.0001 to 10%, preferably from 0.01 to 5%, more preferably from 1 to 3% by total weight ascorbic acid and/or derivatives thereof based on the total weight of the oral care composition.

Mixtures of any of the above described antioxidant vitamins may also be used. Particularly preferred are mixtures of alpha-tocopherol acetate and ascorbyl phosphate (in suitable individual amounts such as those specified above according to the class of antioxidant vitamin).

The total amount of antioxidant vitamin(s) suitably ranges from 0.0001 to 15%, preferably from 0.01 to 5%, more preferably from 2 to 4% by total weight antioxidant vitamin(s) based on the total weight of the oral care composition.

The invention is further illustrated with reference to the following, non-limiting Example.

### EXAMPLE

### Formulations

A toothpaste base formulation was prepared having the ingredients shown in Table 1 below.

All ingredients are expressed by weight percent of the total formulation, and as level of active ingredient.

**Table 1**

| Base Formulation | |
|---|---|
| Ingredient | wt% |
| Sorbitol | 45 |
| Sodium fluoride | 0.32 |
| Sodium saccharin | 0.2 |
| PEG-32 | 2.0 |
| Titanium dioxide | 1.0 |
| Hydrated silica (Tixosil®43, ex Rhodia) | 8 |
| Hydrated silica (Sorbosil®AC77, ex PQ Corp.) | 10 |
| Sodium lauryl sulphate | 1.8 |
| Flavour | 1.2 |
| Carboxymethylcellulose, sodium salt (Aqualon® CMC 9H) | 0.7 |
| Water | to 100 |

In order to prepare test formulations, further ingredients (in a powder format) were added to the above base formulation as specified in Table 2 below.

**Table 2**

| **Test Formulation** | **Composition** |
|---|---|
| **Control** | No additives(Base formulation only) |
| **Comparative Example A** | Base + 1wt% curcumin |
| **Example 1** | Base + 1.4wt% zinc-curcumin complex |
| **Comparative Example B** | Base + 0.5wt% zinc citrate + 1wt% curcumin |
| **Comparative Example C** | Base + 0.5wt% zinc citrate |

### Test Methodology

The test formulations described above were individually evaluated in a pH drop salivary sediment assay. This assay measures the effect of the test formulation on the pH drop (i.e. glycolytic activity) of ex *vivo* oral bacteria. In this assay, stimulated saliva is collected from volunteers first thing in the morning before eating/drinking or oral hygiene. The saliva is centrifuged and washed to yield the sediment which contains large numbers of oral bacteria. The sediment is added to a sugar source and the effects of the test formulation on glycolysis are measured by monitoring pH over time.

### Results

The results of the above evaluation are given below in Table 3.

**Table 3**

| **Test Formulation** | **Acid production over 60 minutes** |
|---|---|
| **Control** | 6.43 |
| **Comparative Example A** | 0.97 |
| **Example 1** | 0.14 |
| **Comparative Example B** | 0.96 |
| **Comparative Example C** | 4.31 |

It can be seen from the results that the formulation according to the invention (Example 1) is the best suppressor of acid production and therefore the best inhibitor of bacterial activity. Notably, Example 1 according to the invention is significantly superior to Comparative Example B, which includes both zinc and curcumin at equivalent levels, but not in the form of a complex.

## Claims

1. A composition for use in a method of suppressing the acid production of microbes found in the oral cavity, in which the oral care composition comprises from 0.2 to 2 wt% of the total composition of a coordination complex in which a metal cation is complexed to one or more ligands which are derived from one or more curcumin compounds in which the curcumin compound is selected from curcumin, demethoxycurcumin, bis-demethoxycurcumin and mixtures thereof.

2. A composition for use according to claim 1 in which the metal cation is a divalent metal cation.

3. A composition for use according to claim 2, in which the metal cation is Zn²⁺.

4. A composition for use according to claim 1, in which the oral care formulation is in the form of a dentifrice or a mouthwash.

## Patentansprüche

1. Zusammensetzung zur Verwendung in einem Verfahren zum Unterdrücken der Säureerzeugung von Mikroben, die in der Mundhöhle gefunden werden, in welcher die Mundpflegezusammensetzung von 0,2 bis 2 Gew.-% der gesamten Zusammensetzung an einem Koordinationskomplex umfasst, in welchem ein Metallkation mit einem oder mehreren Liganden komplexiert ist, die von einer oder mehreren Curcumin-Verbindungen abgeleitet sind, in welchen die Curcumin-Verbindung aus Curcumin, Demethoxycurcumin, Bisdemethoxycurcumin und Mischungen davon ausgewählt ist.

2. Zusammensetzung zur Verwendung nach Anspruch 1, in welcher das Metallkation ein zweiwertiges Metallkation ist.

3. Zusammensetzung zur Verwendung nach Anspruch 2, in welcher das Metallkation Zn²⁺ ist.

4. Zusammensetzung zur Verwendung nach Anspruch 1, in welcher die Mundpflegeformulierung in Form eines Zahnputzmittels oder eines Mundwassers vorliegt.

## Revendications

1. Composition pour une utilisation dans un procédé de suppression de la production d'acide de microbes trouvés dans la cavité orale, dans laquelle la composition pour le soin oral comprend de 0,2 à 2 % en masse de la composition totale d'un complexe de coordination dans lequel un cation de métal est complexé à un ou plusieurs ligands qui sont dérivés d'un ou plusieurs composés de curcumine dans laquelle le composé de curcumine est choisi parmi la curcumine, la déméthoxycurcumine, la bis-déméthoxycurcumine et des mélanges de celles-ci.

2. Composition pour une utilisation selon la revendication 1, dans laquelle le cation de métal est un cation de métal divalent.

3. Composition pour une utilisation selon la revendication 2, dans laquelle le cation de métal est Zn²⁺.

4. Composition pour une utilisation selon la revendication 1, dans laquelle la formulation pour le soin oral est dans la forme d'un dentifrice ou d'un bain de bouche.
